# EUROPEAN PATENT APPLICATION

(11) **EP 2 147 924 A1**
(43) Date of publication of application: **27.01.2010**
(21) Application number: 08161108.9
(22) Date of filing: 24.07.2008
(51) Int. Cl.: C07J 71/00, A61K 31/58, A61P 5/30

(54) **Estratriene derivatives comprising heterocyclic bioisosteres for the phenolic a-ring**

(71) Applicant: Bayer Schering Pharma Aktiengesellschaft, 13353 Berlin (DE)
(72) Inventor: Blume, Thorsten. Dr., 116552 Schildow (DE); Otto, Christiane. Dr., 10178 Berlin (DE); Schmees, Norbert. Dr., 13469 Berlin (DE); Heldmann, Dieter. Dr., 13509 Berlin (DE); Wintermantel, Tim. Dr., 50670 Köln (DE); Kuhnke, Joachim. Dr., 14482 Potsdam (DE)

(57) **Abstract**

The present invention is directed to novel pyrazolo-estrien and triazolo-estrien-derivatives, pharmaceutical compositions containing them and their use in the treatment or prevention of disorders and diseases mediated by an estrogen receptor such as hot flashes, vaginal dryness, osteopenia, osteoporosis, hyperlipidemia, loss of cognitive function, degenerative brain diseases, cardiovascular diseases, cerebrovascular diseases, hormone sensitive cancers and hyperplasia (in tissues including breast, endometrium, and cervix in women and prostate in men), endometriosis, uterine fibroids, osteoarthritis; and as contraceptive agents either alone or in combination with a progestogen or progestogen antagonist. The compounds of the invention are selective estrogen receptor modulators.

## Description

The present invention is directed to novel pyrazolo-estrien and triazolo-estrien-derivatives, pharmaceutical compositions containing them and their use in the treatment or prevention of disorders and diseases mediated by an estrogen receptor such as hot flashes, vaginal dryness, osteopenia, osteoporosis, hyperlipidemia, loss of cognitive function, degenerative brain diseases, cardiovascular diseases, cerebrovascular diseases, hormone sensitive cancers and hyperplasia (in tissues including breast, endometrium, and cervix in women and prostate in men), endometriosis, uterine fibroids, osteoarthritis; and as contraceptive agents either alone or in combination with a progestogen or progestogen antagonist. The compounds of the invention are selective estrogen receptor modulators.

Estrogens are a group of female hormones essential for the reproductive process and for the development of the uterus, breasts, and other physical changes associated with puberty. Estrogens have an effect on various tissues throughout a woman's body, not only those involved in the reproductive process, such as the uterus, breasts, and external genitalia, but also tissues in the central nervous system, bones, the liver, skin, and the urinary tract. The ovaries produce most of the estrogens in women's body. Endogenous estrogens, such as 17beta-estradiol and estrone, play a central role in the development of and maintenance of the female sex organs, mammary glands, and other sexual characteristics. In addition to their role as female sex hormone, estrogens are involved in the growth and function of a number of other tissues, such as the cardiovascular system, the central nervous system, and the skeleton, both in females and males. The significance of the estrogens in the development of the female reproductive system led to the development of a variety of compounds that interact with the estrogen receptors, such as contraceptives and agents for treatment of breast cancers.

Combined oral contraceptive pills (COCPs) were developed to prevent ovulation by suppressing the release of gonadotropins. Combined hormonal contraceptives, including COCPs, inhibit follicular development and prevent ovulation as their primary mechanism of action (Trussell, James (2007). "Contraceptive Efficacy", in Hatcher, Robert A., et al: Contraceptive Technology, 19th rev. ed., New York: Ardent Media.; Speroff, Leon; Darney, Philip D. (2005). "Oral Contraception", A Clinical Guide for Contraception, 4th ed., Philadelphia: Lippincott Williams & Wilkins, pp. 21-138; Loose, Davis S.; Stancel, George M. (2006). "Estrogens and Progestins", in Brunton, Laurence L.; Lazo, John S.; Parker, Keith L. (eds.): Goodman & Gilman's The Pharmacological Basis of Therapeutics, 11th ed., New York: McGraw-Hill, pp. 1541-1571; Glasier, Anna (2006). "Contraception", in DeGroot, Leslie J.; Jameson, J. Larry (eds.): Endocrinology, 5th edition, Philadelphia: Elsevier Saunders, pp. 2993-3003; Rivera R, Yacobson I, Grimes D (1999). "The mechanism of action of hormonal contraceptives and intrauterine contraceptive devices". Am J Obstet Gynecol 181 (5 Pt 1): 1263-9).

Progestagen negative feedback decreases the pulse frequency of gonadotropin-releasing hormone (GnRH) release by the hypothalamus, which decreases the release of follicle-stimulating hormone (FSH) and greatly decreases the release of luteinizing hormone (LH) by the anterior pituitary. Decreased levels of FSH inhibit follicular development, preventing an increase in estradiol levels. Progestagen negative feedback and the lack of estrogen positive feedback on LH release prevent a mid-cycle LH surge. Inhibition of follicular development and the absence of a LH surge prevent ovulation (Trussell, James (2007). "Contraceptive Efficacy", in Hatcher, Robert A., et al: Contraceptive Technology, 19th rev. ed., New York: Ardent Media.; Speroff, Leon; Darney, Philip D. (2005). "Oral Contraception", A Clinical Guide for Contraception, 4th ed., Philadelphia: Lippincott Williams & Wilkins, pp. 21-138; Loose, Davis S.; Stancel, George M. (2006). "Estrogens and Progestins", in Brunton, Laurence L.; Lazo, John S.; Parker, Keith L. (eds.): Goodman & Gilman's The Pharmacological Basis of Therapeutics, 11th ed., New York: McGraw-Hill, pp. 1541-1571).

Estrogen was originally included in oral contraceptives for better cycle control (to stabilize the endometrium and thereby reduce the incidence of breakthrough bleeding), but was also found to inhibit follicular development and help prevent ovulation. Estrogen negative feedback on the anterior pituitary greatly decreases the release of FSH, which inhibits follicular development and helps prevent ovulation (Trussell, James (2007). "Contraceptive Efficacy", in Hatcher, Robert A., et al: Contraceptive Technology, 19th rev. ed., New York: Ardent Media.; Speroff, Leon; Darney, Philip D. (2005). "Oral Contraception", A Clinical Guide for Contraception, 4th ed., Philadelphia: Lippincott Williams & Wilkins, pp. 21-138; Loose, Davis S.; Stancel, George M. (2006). "Estrogens and Progestins", in Brunton, Laurence L.; Lazo, John S.; Parker, Keith L. (eds.): Goodman & Gilman's The Pharmacological Basis of Therapeutics, 11th ed., New York: McGraw-Hill, pp. 1541-1571).

Although the oral contraceptives are highly effective, their use is associated with unpleasant side effects (such as nausea, depression, weightgain, and headache) and an increased long-time risk of severe disease (such as thromboembolism, stroke, myocardial infarction, hepatic adenoma, gall bladder disease, and hypertension). Bleeding irregularities (such as breakthrough bleeding, spotting, and amenorrhea) are also frequent. A progestin, when administered alone, causes an increased incidence of changes in menstrual patterns, especially a marked increase in the amount and duration of menstrual bleeding.

The estrogen receptor beta (ER beta) was discovered as a second subtype of the estrogen receptor (Kuiper et al. (1996), Proc. Natl. Acad. Sci. 93: 5925-5930; Mosselman, Dijkema (1996) Febs Letters 392: 49-53; Tremblay et al. (1997), Molecular Endocrinology 11: 353-365). Intensive efforts are being made to investigate the distribution of ER alpha and ER beta in several tissues.

The estrogen receptor alpha (ER alpha) is expressed in neurons projecting to GnRHpositive neurons in the hypothalamus and is required for mediating the positive estradiol feedback leeding to the preovulatory LH surge. On the other hand, estrogen receptor alpha in the pituitary and in the hypothalamus is involved in mediating the negative feedback of estradiol which leads to suppression of LH/FSH secretion. Estrogen receptor alpha activation is important for the induction of progesterone receptor expression in some reproductive organs such as the uterus and the hypothalamus. In other words, estrogen receptor activation is a prerequisite for progestin action. Estrogen receptor alpha mediates estrogenic responses in the uterus (stimulation of epithelial cell proliferation), in the mammary gland (stimulation of epithelial cell proliferation), the bone (prevention of osteoblast apoptosis) and the brain (prevention of hot flushes). The primary role of the estrogenic component in combined oral contraception is the maintainance of a regular bleeding pattern during the pill-free days. The contraceptive function, i.e. ovulation inhibition is primarily mediated by the gestagenic component. However, ovulation inhibition leads to suppression of endogenous estradiol levels which would cause hot flushes and bone loss in young women. Addition of estrogens in combined oral contraception prevents these symptoms. Moreover, the estrogenic component is required to induce the progesterone receptor and to enable the contraceptive action of the gestagenic component. In other words, without estrogen addition, much higher doses of progestins would be required to induce ovulation inhibition.

Intensive efforts have focused on the selective estrogen receptor modulators for treatment and prevention of postmenopausal conditions, such as osteoporosis, coronary artery disease, depression and Alzheimer disease.
Menopause is defined as the permanent cessation of menses due to loss of ovarian follicular function and the almost termination of estrogen production. The midlife transition of menopause is characterized by a decrease in estrogen that provokes both short-term and long-term symptoms with the vasomotor, urogenital, cardiovascular, and skeletal and central nervous systems, such as hot flushes, urogenital atrophy, increased risk of cardiovascular disease, osteoporosis, cognitive and psychological impairment, including an increased risk of cognitive disorders and Alzbeimer's disease (AD). All these menopausal symptoms can be treated successfully with estrogen. Since estradiol stimulates uterine epithelial cell proliferation and thus increases the risk for endometrial carcinoma, the addition of progestins is required in postmenopausal women that still have an uterus. Progestins inhibit the estradiol activated uterine epithelial cell proliferation.
Seventy-five percent of all women experience some occurrence of vasomotor symptoms associated with the onset of menopause such as body sweating and hot flushes. These complaints may begin several years before menopause and in some women may continue for more than 10 years either relatively constant or as instant attacks without a definable, provoking cause.
Urogenital symptoms associated with the onset of menopause involving the vagina include a sensation of dryness, burning, itching, pain during intercourse, superficial bleeding and discharge, along with atrophy and stenosis. Symptoms involving the urinary tract include a burning sensation during urination, frequent urgency, recurrent urinary tract infections, and urinary incontinence. These symptoms have been reported to occur in up to 50% of all women near the time of menopause and are more frequent a few years after menopause. If left untreated, the problems can become permanent. Heart attack and stroke are major causes of morbility and mortality among senior women. Female morbility from these diseases increases rapidly after menopause. Women who undergo premature menopause are at greater coronary risk than menstruating women of similar age. The presence of serum estrogen has a positive effect on serum lipids. The hormone promotes vasodilation of blood vessels, and enhances the formation of new blood vessels. Thus the decrease in serum estrogen levels in postmenopausal women results in adverse cardiovascular effect. Additionally, it is theorized that differences in the ability of blood to coagulate may account for the observed difference in the occurrence of heart disease before and after menopause.
The skeleton is under a continuous process of bone degeneration and regeneration in a carefully regulated interaction among the bone cells. These cells are directly affected by estrogen. Estrogen deficiency results in a loss of bone structure and a decrease of bone strength. Rapid loss of bone mass during the year immediately following menopause leads to postmenopausal osteoporosis and increased risk of fracture.
Estrogen deficiency is also one of the causes for the degenerative changes in the central nervous system and may lead to Alzheimer's disease and decline of cognition. Recent evidence suggests an association between estrogen, menopause and cognition. More particularly, it has been reported that estrogen replacement therapy and the use of estrogen in women may prevent the development of Alzheimer disease and improve cognitive function.
Hormone replacement therapy (HRT) - more specifically estrogen replacement therapy (ERT) - is commonly prescribed to address the medical problems associated with menopause, and also to help hinder osteoporosis and primary cardiovascular complications (such as coronary artery disease) in both a preventive and therapeutical manner. As such, HRT is considered as a medical therapy for prolonging the average life span of postmenopausal women and providing a better quality of life.
ERT effectively relieves the climacteric symptoms and urogenital symptoms and has shown significant benefits in the prevention and treatment of heart disease in postmenopausal women. Clinical reports have shown that ERT lowered heart attack rates and mortality rates in populations that received ERT versus similar populations not on ERT. ERT initiated soon after menopause may also help maintain bone mass for several years. Controlled investigations have shown that treatment with ERT has a positive effect even in older women up to age of 75 years.
However, as mentioned above, there are numerous undesirable effects associated with ERT that reduce patient compliance. Venous thromboembolism, gallbladder disease, resumption of menses, mastodynia and a possible increased risk of developing uterine and/or breast cancer are the risks associated with ERT. Up to 30% of women who were prescribed ERT did not fill the prescription, and the discontinuation rate is between 38% and 70%, with safety concerns and adverse effects (bloating and break-through bleeding) the most important reasons for discontinuation.

WO 2004/005314 describes novel estrieno[3.2-b]/[3,4-c]pyrrole derivatives, pharmaceutical compositions containing them and their use in the treatment or prevention of disorders and diseases mediated by an estrogen receptor such as hot flashes, vaginal dryness, osteopenia, osteoporosis, hyperlipidemia, loss of cognitive function, degenerative brain diseases, cardiovascular diseases, cerebrovascular diseases, hormone sensitive Cancers and hyperplasia (in tissues including breast, endometrium, and cervix in women and prostate in men), endometriosis, uterine fibroids, osteoarthritis; and as contraceptive agents either alone or in combination with a progestogen or progestogen antagonist. The claimed compounds are selective estrogen receptor modulators, but relatively weak Estrogen Receptor ligands.

WO 2007/089291 discloses non-steroidal pyrazol derivatives which are useful for treating or preventing a variety of conditions related to estrogen functioning, especially for eliciting an estrogen receptor modulating effect in a mammal in need thereof. The described compounds are also relatively weak Estrogen Receptor agonists.

For combined contraception Ethinylestradiol is the current market standard. However, the administration of Ethinylestradiol is associated with an increased risk for venous thrombembolism and a high inter- and intraindividual variability of the bioavailability.

It is therefore an object of the present invention to provide further compounds which are estrogen receptor agonists. These compounds should have an oral bioavailability at least comparable to Ethinylestradiol.

The object is achieved according to the present invention by the provision of compounds of the general formula (I) 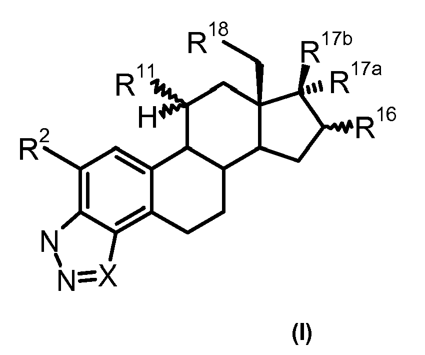 wherein
- X: is selected from the group consisting of nitrogen and CR^{a}, whereby
- R^{a}: stands for hydrogen, C₁₋₃-alkyl group, a CₚF₂ₚ₊₁ group with p = 1-3,
- R²: represents a hydrogen atom, a halogen atom, a C₁₋₃-alkyl group or a trifluoromethyl group,
- R¹¹: is selected from the group of hydrogen, halogen, C₁₋₃-alkyl, C₂₋₃-alkenyl, C₂₋₃-alkinyl and C₁₋₃-alkoxy,
- R¹⁶: is selected from the group consisting of hydrogen, hydroxyl, halogen, C₁₋₃-alkyl, C₂₋₃-alkenyl, C₂₋₃-alkinyl and trifluoromethyl,
- R^{17a} and R^{17b}: stand for a hydrogen atom, a hydroxyl group, an optionally substituted C₁₋₃-alkyl group, an optionally substituted C₂₋₃-alkenyl group, an optionally substituted C₂₋₃-alkinyl group, a halogen atom, a group -OCOR^{b}, whereby
- R^{b}: represents a group -(CH₂)ₙCOOH with n = 2 or 3, or a C₁₋₅-alkyl group with the proviso if R^{17a} stands for a hydroxyl group, R^{17b} represents a hydrogen atom or an optionally substituted C₁₋₃-alkyl group, an optionally substituted C₂₋₃-alkenyl group or a group -OCOR^{b} with the definition for R^{b} as mentioned above, and vice versa, or
- R^{17a} and R^{17b}: stand together for an oxygen atom,
and
- R¹⁸: represents a hydrogen atom or a methyl group,
or a pharmaceutically acceptable salt thereof.

The compounds according to the present invention show a comparable oral bioavailability as Ethinylestradiol.
Additionally, the compounds of the present invention also have considerably reduced hepatic estrogenicity compared to the current market standard ethinyl estradiol.
Preferably, the compounds according to the present invention exhibit agonistic activity on the Estrogen Receptor (ER) in the uterus, in the bone and brain as well as in the breast like ethinyl estradiol and estradiol. Therefore, they are suitable for oral contraception.
Therefore, use of the compounds of the present invention for contraception alone or in combination with an additional active ingredient is an embodiment of this invention.

The ER agonistic activity in the uterus allows a sufficient bleeding control which is desirable for a contraception method.
The ER agonistic activity in the bone and brain is preferred in order to prevent young women which are taking combined oral contraception from bone loss and hot flushes. Additionally, the compounds of the invention are suitable for the treatment and prevention of decreasing systemic estrogen levels. Preferably the compounds according to the invention are suitable for ERT, especially for the treatment and prevention of vasomotor, urogenital and cognitive disorders

Illustrative of the invention is a pharmaceutical composition which comprises at least one compound described in formula (I) and optionally at least one pharmaceutically suitable excipient and/or carrier. A further embodiment of the present invention is a pharmaceutical composition comprising compounds of the general formula I and, optionally at least one additional active ingredient. According to the present invention the additional active ingredient is a SERM (selective estrogen receptor modulator) or a SERD (selective estrogen receptor destabilizer) or a progestogen.

An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.
Exemplifying the invention are methods of treating a disorder mediated by one or more estrogen receptors in a subject in need thereof comprising administering to the subject a therapeutically effective amount of any of the: compounds or pharmaceutical compositions described above.

Illustrating the invention is a method of contraception comprising administering to a subject in need thereof co-therapy with an effective amount of any of the compounds described herein with a progestogen. Progestogens which are useful for such co-therapy are progesterone, trimegestone, medroxyprogesterone acetate, megestrol acetate, cyproterone acetate, chlormadinone acetate, nestorone, levonorgestrel, norgestimate, desogestrel, ethonogestrel (3-Ketodesogestrel), nomegestrol acetate (NOMAC), norethisterone acetate (NETA), drospirenone, gestodene, dienogest, norethindrone acetate, danazole, norgestrel, and tanaproget.

Another example of the invention is the use of any of the compounds described herein in the preparation of a medicament for treating: (a) hot flashes, (b) vaginal dryness, (c) osteopenia, (d) osteoporosis, (e) hyperlipidemia, (f) loss of cognitive function, (g) a degenerative brain disorder, (h) a cardiovascular disease, (i) a cerebrovascular disease (j) breast cancer, (k) endometrial cancer, (I) cervical cancer, (m) prostate cancer, (n) benign prostatic hyperplasia, (o) endometriosis, (p) uterine fibroids, (q) osteoarthritis and for (r) contraception in a subject in need thereof. For these indications the compounds according to the invention can likewise be used in combination with any given Selective Estrogen Receptor Destabilizer (SERD) or Selective Estrogen Receptor Modulator (SERM) or with a progestogen.
Suitable for combination with the compounds according to the invention in this connection are for example the following fulvestrant and compounds claimed in WO98/007740, WO03/045971 and WO01/00652 (SERDs) as well as Tamoxifen, Raloxifen, Bazedoxifen, Arzoxifen, Lasofoxifen, Clomiphene, Ormeloxifene, Levormeloxifene, Toremifene, Ospemifene, TAS-108, PSK-3471, CHF-4227, GSK-2329802, LY-2066948 and the compounds claimed in WO01/68634 and WO 03/033461 (SERMs) as well as progesterone, trimegestone, medroxyprogesterone acetate, megestrol acetate, cyproterone acetate, chlormadinone acetate, nestorone, levonorgestrel, norgestimate, desogestrel, ethonogestrel (3-Ketodesogestrel), nomegestrol acetate (NOMAC), norethisterone acetate (NETA), drospirenone, gestodene, dienogest, norethindrone acetate, danazole, norgestrel, and tanaproget.(progestogens).

The present invention is preferably directed to compounds of formula (I) 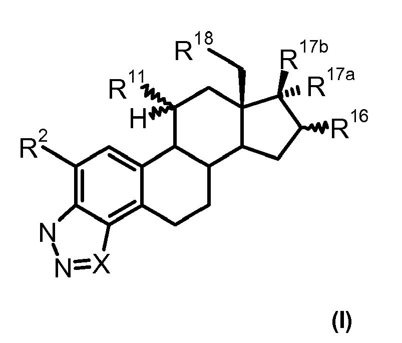 wherein X, R², R¹¹, R¹⁶, R^{17a} and R^{17b} are as herein defined.

As mentioned above, the compounds of the present invention are modulators of the estrogen receptor alpha and hence useful for the treatment and prevention of disorders associated with estrogen depletion, including, but not limited to hot flashes, vaginal dryness, osteopenia, osteoporosis, hyperlipidemia, loss of cognitive function, degenerative brain diseases, cardiovascular diseases and cerebrovascular diseases; for the treatment of hormone sensitive cancers and hyperplasia (in tissues including breast, endometrium, and cervix in women and prostate in men); for the treatment and prevention of endometriosis, uterine fibroids, and osteoarthritis.

The compounds of the present invention are especially suitable as contraceptive agents either alone or in combination with a progestogen. Examples for useful progestogens are mentioned above.

The substituents, defined as groups, of the compounds according to the invention of the general formula (I) may in each case have the following meanings:

C₁-₃- and C₁-₅-alkyl group means unbranched or optionally branched alkyl radicals. Examples thereof are a methyl, ethyl, n-propyl, isopropyl, n-, iso-, tert-butyl, an n-pentyl, 2,2-dimethylpropyl, 3-methylbutyl group.
In the meaning of R^{17a} and R^{b}, the methyl group is preferred.
In the meaning of R^{17a} an optionally substituted C₁-₃-alkyl group means methyl, ethyl, propyl and iso-propyl.

Alkenyl means unbranched or optionally branched alkenyl radicals. Examples of the meaning of a C₂-₃-alkenyl group in the context of the invention are vinyl, propenyl and allyl. The vinyl group is preferred,

Alkynyl means unbranched or optionally branched alkynyl radicals. A C₂-₃-alkynyl radical is intended to be for example an ethynyl, propynyl, but preferably an ethynyl group.

Suitable for a partly or completely fluorinated C₁-₃-fluoroalkyl group is in particular the trifluoromethyl group.

A halogen atom may be a fluorine, chlorine, bromine or iodine atom. Fluorine is preferred here.

For the compounds of the present invention, each R¹¹ as well as R¹⁶ substituent may be in an alpha-or a beta-orientation.

For the combined oral contraception any of the compounds described herein have to be combined with a progestogen like progesterone, trimegestone, medroxyprogesterone acetate, megestrol acetate, cyproterone acetate, chlormadinone acetate, nestorone, levonorgestrel, norgestimate, desogestrel, ethonogestrel (3-Ketodesogestrel), nomegestrol acetate (NOMAC), norethisterone acetate (NETA), drospirenone, gestodene, dienogest, norethindrone acetate, danazole, norgestrel, and tanaproget.

As used herein, the term "disease or disorder modulated or mediated by an estrogen receptor" shall mean any disease or disorder which is mediated by the estrogen receptor alpha, any disease or disorder which is mediated by the estrogen receptor beta or any disease or disorder which is mediated by both the estrogen receptors alpha and beta. For example, hot flashes, vaginal dryness, osteopenia, osteoporosis, hyperlipidemia, loss of cognitive function, a degenerative brain disorder, cardiovascular disease, cerebrovascular disease, breast cancer, endometrial cancer, cervical cancer, prostate cancer, benign prostatic hyperplasia (BPH), endometriosis, uterine fibroids, osteoarthritis and contraception.
As used herein, the term "degenerative brain disease" shall include cognitive disorder, dementia (regardless of underlying cause) and Alzheimer's disease. As used herein, the term "cardiovascular disease" shall include elevated blood lipid levels, coronary arteriosclerosis and coronary heart disease.
As used herein, the term "cerebrovascular disease" shall include abnormal regional cerebral blood flow and ischemic brain damage.

The term "subject" as used herein, refers to an animal, preferably a mammal, most preferably a human, who is the object of treatment, observation or experiment. The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. Wherein the present invention is directed to co-therapy comprising administration of one or more compound(s) of formula (I) and a progestogen, "therapeutically effective amount" shall mean that amount of the combination of agents taken together so that the combined effect elicits the desired biological or medicinal response. For example, the therapeutically; effective amount of co-therapy comprising administration of a compound of formula (I) and progestogen or SERM or SERD would be the amount of the compound of formula (I) and the amount of the progestogen, SERM or SERD that when taken together or sequentially have a combined effect that is therapeutically effective. Further, it will be recognized by one skilled in the art that in the case of co-therapy with a therapeutically effective amount, as in the example above, the amount of the compound of formula I and/or the amount of the progestogen or SERM or SERD individually may or may not be therapeutically effective.
As used herein, the term "co-therapy" shall mean treatment of a subject in need thereof by administering one or more compounds of formula (I) with a progestogen or SERM or SERD, wherein the compound(s) of formula (I) and the progestogen, SERM or SERD are administered by any suitable means, simultaneously, sequentially, separately or in a single pharmaceutical formulation. Where the compound(s) of formula (I) and the progestogen, SERM or SERD are administered in separate dosage forms, the number of dosages administered per day for each compound may be the same or different. The compound(s) of formula (I) and the progestogen, SERM or SERD may be administered via the same or different routes of administration. Examples of suitable methods of administration include, but are not limited to, oral, intravenous (iv.), intramuscular (im.), subcutaneous (sc.), transdermal, and rectal. Compounds may also be administered directly to the nervous system including, but not limited to, intracerebral, intraventricular, intracerebroventricular, intrathecal, and intracisternal, intraspinal and / or pert-spinal routes of administration by delivery via intracranial or intravertebral needles and / or catheters with or without pump devices. The compound(s) of formula (I) and the progestogen or SERM or SERD may be administered according to simultaneous or alternating regimens, at the same or different times during the course of the therapy, concurrently in divided or single forms.
As used herein, the term "composition" is intended to encompass a product comprising the specified ingredients in the specified amounts, as well as any product which results, directly or indirectly, from combinations of the specified ingredients in the specified amounts.
For use in medicine, the salts of the compounds of this invention refer to non-toxic "pharmaceutically acceptable salts." Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts. Thus, representative pharmaceutically acceptable salts include the following: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methyinitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

In an embodiment of the present invention R^{17a} is selected from the group consisting of hydrogen, an optionally substituted C₁₋₃-alkyl group, an optionally substituted C₂₋₃-alkenyl group, an optionally substituted C₂₋₃-alkinyl group, whereas R^{17b} is selected from the group consisting of hydroxyl, fluorine, -OCOR^{b} with R^{b} as defined above.

In an embodiment of the present invention R¹⁶ is selected from the group consisting of hydrogen, hydroxyl and fluorine.

In an embodiment of the present invention R¹⁸ is a hydrogen atom.

In an embodiment of the present invention X stands for CR^{a} with R^{a} as defined above.

In another embodiment of the present invention R^{a} stands for a hydrogen atom, a group trifluoromethyl or a methyl group.

In another embodiment of the present invention R^{17a} is selected from the group consisting of hydrogen, methyl, trifluoromethyl, vinyl and ethinyl.

In another embodiment of the present invention R^{17a} represents a hydroxyl group or a fluorine atom.

In a further embodiment of the present invention R¹¹ represents a hydrogen atom, a fluorine atom, a hydroxyl group or a methoxy group.

In a further embodiment of the present invention R² stands for a hydrogen atom, a fluorine atom or a trifluoromethyl group.

In a further embodiment of the present invention R¹⁶ represents a hydroxyl group, R^{17a} a hydrogen atom and R^{17b} a fluorine atom.

In a further embodiment of the present invention R^{17b} represents a hydroxyl group, R^{17a} a hydrogen atom, a vinyl, ethinyl, methyl or a trifluoromethyl group and R¹⁶ a hydrogen or fluorine atom or a hydroxyl group.

The compounds mentioned below, and the uses thereof, are preferred according to the invention:
2'H-Pyrazolo[3',4':3,4]estra-1,3,5(10)-trien-17β-ol
17α-Methyl-2'H-pyrazolo[3',4':3,4]estra-1,3,5(10)-trien-17β-ol
17α-Ethyl-2'H-pyrazolo[3',4':3,4]estra-1,3,5(10)-trien-17β-ol
17α-Propyl-2'H-pyrazolo[3',4':3,4]estra-1,3,5(10)-trien-17β-ol
17α-Vinyl-2'H-pyrazolo[3',4':3,4]estra-1,3,5(10)-trien-17β-ol
17α-Ethinyl-2'H-pyrazolo[3',4':3,4]estra-1,3,5(10)-trien-17β-ol
2'H-Pyrazolo[3',4':3,4]estra-1,3,5(10)-trien-17-one
2-Fluoro-2'H-pyrazolo[3',4':3,4]estra-1,3,5(10)-trien-17β-ol
2-Fluoro-17α-methyl-2'H-pyrazolo[3',4':3,4]estra-1,3,5(10)-trien-17β-ol
17α-Ethyl-2-fluoro-2'H-pyrazolo[3',4':3,4]estra-1,3,5(10)-trien-17β-ol
2-Fluoro-17α-propyl-2'H-pyrazolo[3',4':3,4]estra-1,3,5(10)-trien-17β-ol
2-Fluoro-17α-vinyl-2'H-pyrazolo[3',4':3,4]estra-1,3,5(10)-trien-17β-ol
17α-Ethinyl-2-fluoro-2'H-pyrazolo[3',4':3,4]estra-1,3,5(10)-trien-17β-ol
11β-Fluoro-2'H-Pyrazolo[3',4':3,4]estra-1,3,5(10)-trien-17β-ol
11β-Fluoro-17α-Methyl-2'H-pyrazolo[3',4':3,4]estra-1,3,5(10)-trien-17β-ol
17α-Ethyl-11β-fluoro-2'H-pyrazolo[3',4':3,4]estra-1,3,5(10)-trien-17β-ol
11β-Fluoro-17α-Propyl-2'H-pyrazolo[3',4':3,4]estra-1,3,5(10)-trien-17β-ol
11β-Fluoro-17α-Vinyl-2'H-pyrazolo[3',4':3,4]estra-1,3,5(10)-trien-17β-ol
17α-Ethinyl-11β-fluoro-2'H-pyrazolo[3',4':3,4]estra-1,3,5(10)-trien-17β-ol
5'-Methyl-2'H-pyrazolo[3',4':3,4]estra-1,3,5(10)-trien-17β-ol
5',17-Dimethyl-2'H-pyrazolo[3',4':3,4]estra-1,3,5(10)-trien-17β-ol
2-Fluoro-5',17-dimethyl-2'H-pyrazolo[3',4':3,4]estra-1,3,5(10)-trien-17β-ol
2-Fluoro-5'-Methyl-2'H-pyrazolo[3',4':3,4]estra-1,3,5(10)-trien-17β-ol

The compounds may additionally exist as diastereomers. It is to be understood that all such isomers and mixtures thereof are encompassed within the scope of the present invention.
Furthermore, some of the crystalline forms for the compounds may exist as polymorphs and as such are intended to be included in the present invention.
In addition, some of the compounds may form solvates with water (i.e. hydrates) or common organic solvents, and such solvates are also intended to be encompassed within the scope of this invention.
Where the processes for the preparation of the compounds according to the invention give rise to mixture of stereoisomers, these isomers may be separated by conventional techniques such as preparative chromatography.
The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.
During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.
The present invention includes within its scope prodrugs of the compounds of this invention. In general, such prodrugs will be functional derivatives of the compounds which are readily convertible in vivo into the required compound. Thus, in the methods of treatment of the present invention, the term "administering" shall encompass the treatment of the various disorders described with the compound specifically disclosed or with a compound which may not be specifically disclosed, but which converts to the specified compound in vivo after administration to the patient. Conventional procedures for the selection and preparation of suitable prodrug derivatives are described, for example, in "Design of Prodrugs", ed. H. Bundgaard, Elsevier, 1985.
The utility of the compounds of the instant invention to treat disorders mediated by an estrogen receptor may be determined according to the procedures described below:

### Biological Characterization of the Compounds According to the Invention

### Estrogen Receptor Binding Studies

### Binding to estrogen receptor α

This assay monitors the binding of tool compounds to the estrogen receptor α (ERα) using competition experiments with radioactively labeled estradiol. The ERα protein used in the binding assay was purified from cytosolic fractions of Hi5 cells transfected with recombinant baculoviruses encoding either the human ERα. Aliquots of cytosolic fractions were stored at -80°C and had protein concentrations of 5 - 7 mg/ml (as determined by the BCA method).
The binding assay was performed in Greiner microtiter plates with conical wells. 5 µl of the test compound (various concentrations dissolved in 10% DMSO) were mixed with 15 µl of 16.66 nM ³H-estradiol ([2,4,6,7-³H(N)-estradiol, 70 - 115 Ci/mmol, NEN) in assay buffer (10 mM TIS/HCl pH7.4, 1.5 mM EDTA, 10% glycerol).
30 µl of cytosol were added leading to a final volume of 50 µl per well. Final protein concentration was 50 - 200 µg per well, final cold estradiol or tool compound concentration ranged from 0.3 nM to 1 µM. All samples were tested in duplicate. Unspecific binding was determined in the presence of 10 µM cold estradiol, total binding was measured in the absence of cold estradiol or test compound. Incubation of ERα with the different concentrations of estradiol or tool compound in the presence of radioactively labeled estradiol was performed for 1 h at room temperature. Afterwards, 45 µl of the incubation mixture were transferred to microtiter filtration plates preloaded with 50 µl per well cold charcoal suspension (2% charcoal, 0.2% dextran T70 in 10 mM TRIS/HCl pH 7.4, 1.5 mM EDTA, 15% glycerol) (EVENT plates, 0.2 µm pores, low protein binding filters by Eppendorf) to bind non-receptor bound radioactivity. The mixtures were filtered into Picoplates using a vacuum pump to separate protein-bound from unbound radioactive estradiol. The protein-bound radioactivity was measured by addition of 200 µl Microszint-40 (Canberra Packard) to each well using a TopCount scintillation counter. Dose-response curves were generated and the IC₅₀ values for estradiol and the test compounds were calculated. In addition, KF values were determined by dividing the IC₅₀ of the test compound by the IC₅₀ of the reference (i.e. estradiol). By definition, the KF of estradiol is 1.

### Transactivation assay in U2OS cells

To analyse the transactivational properties of test compounds on ERα we used U2OS cells (a human osteosarcoma cell line) transiently transfected with either ERα and an estrogen-responsive luciferase reporter plasmid, i.e. p(ERE)₂-luc⁺ as described previously by Wessler et al., J Steroid Biochem Mol Biol. (2006), 98(1):25-35. Cells were serum-starved for at least 24 hours and seeded in 96 well plates at a density of 10000 cells per well in phenolred-free DMEM containing 5% charcoal stripped serum, 4 mM glutamine, 100 U/ml penicillin, and 100 µg/ml streptomycin. 6 hours after seeding cells were transiently transfected overnight with the luciferase reporter plasmid and the appropriate human estrogen receptor using FuGENE 6 according to the instructions of the manufacturer. The next day the medium was removed and 180 µl of DMEM containing 5% charcoal-stripped were added to the cells.
Serial dilutions of test compounds and estradiol were prepared in 1 % DMSO ranging from 10⁻⁷ - 10⁻¹² M. 20 µl of these dilutions were added to the cells for 24 hours, leading to final concentrations of 10⁻⁸ - 10⁻¹³ M of estradiol or test compound. respectively. After stimulation of the cells with either vehicle or test compounds, the medium was aspirated and cells were lysed with 30 µl of 1x Lysis Reagent (Promega E1531) at room temperature for 30 minutes. Subsequently 30 µl of luciferase substrate A ( PharMingen 556867) and 30 µl of luciferase substrate B (PharMOngen 556869) were added. Plates were measured in a luminometer (DYNATECH). Dose response curves were generated and ED₅₀ values were calculated using Sigma Plot.

### Transactivation assay in stably transfected MCF-7 cells (MVLN assay)

To analyse activation of the ERα by diverse tool compounds in a second cellular system, we used MCF-7 cells endogenously expressing ERα and stably transfected with the estrogen-responsive vitellogenin-tk-luciferase reporter plasmid. Cells were starved for at least 3 days in phenolred-free DMEM containing 5% charcoal-stripped serum, 4 mM glutamine, 100 U/ml penicillin and 100 µg/ml streptomycin. 6000 cells were seeded in 25 µl medium per well on a 384 well plate and stimulated for 24 hours with either vehicle, estradiol or test compounds (concentration range as 10⁻⁶ - 10⁻¹³ M). Luciferase activity was measured after addition of 25 µl steady-Glo in a TopCount. Dose response curves were generated and EC₅₀ values were calculated using Sigma plot.

### Induction of alkaline phosphatase in human endometrial Ishikawa cells

Alkaline phosphatase is an estrogen target gene in the normal uterine epithelium as well as in endometrial-derived cancer cells, such as Ishikawa cells. The induction of alkaline phosphatase activity by estradiol or test compounds can be used to assess the in vitro potency of test compounds on endogenously exppressed ERα in these cells. Ishikawa cells (from European collection of cell cultures) were maintained in phenolred-free MEM with 5% FCS, 4 mM glutamine, 1% non essential amino acids and 100 U / ml penicillin and 100 µg/ml streptomycin. Prior to testing, cells were starved for 72 hours in phenolred-free MEM containing 5% charcoal-stripped FCS. Vehicle or test compounds were added to the culture media at varying concentrations covering a range from 10⁻⁷ to 10⁻¹³ M. Cells are incubated for 72 hours. On the third day, the media were removed; cells were washed two times with 50 µl PBS and then frozen at -80°C for 20 min. After thawing at room temperature for 10 min, cells were incubated with 100 µl of 1-Step PNPP reagent (Pierce) and incubated for 1.5 h at room temperature. OD was measured at 405 nm sing a Polarstar Optima. Dose response curves were generated using and ED₅₀ values were calculated using Sigma plot.

### Stimulation of uterine growth in vivo

One classical ERα-mediated in vivo action is the stimulation of uterine growth in castrated animals. To assess the in vivo potency of test compounds at ERα, adult female Wistar rats were ovariectomized. 14 days after ovariectomy, animals were treated daily subcutaneously for 14 days with either vehicle (benzylbenzoate/ricinusoil 1+4) or different doses of the test compounds. Animals were sacrificed on day 15 and the relative uterine weights were determined. Dose response curves were generated in Sigma plot to determine the in vivo activity of the test compounds at ERα in comparison to estradiol as standard.

### Stability in Hepatocytes

The metabolic stability of test compounds with respect to phase 1 and phase 2 metabolisms was investigated by incubation of the compounds with hepatocytes. These investigations were performed for characterisation of compounds in case of phase 2 metabolism is to be expected. The different test compounds were incubated for a sufficient period of time with a suspension of human cryopreserved hepatocytes of comparable metabolic activity (no. of hepatocytes in homogenous suspension). After analytical workup the concentrations of the test compounds after different incubation periods were related to the initial concentration of the respective test compound (sero hour's time point). The resulting area under the concentration-time-curve of the test compound investigated in the assay is used to derive the respective Fmax value. This data gives the maximal theoretical bioavailability of the test compound investigated.

### Results

Compounds of the present invention were tested according to the procedures described above for binding to ERα for transactivation at ERα for activity in the MVLN assay, for induction of alkaline phosphatase in Ishikawa cells, and for in vivo activity with regard to stimulation of uterine growth.
As can be seen in Table 1 exemplary for, but not limited to compound 1, the compounds of the present invention are potent ERalpha agonists in vitro regarding binding and transactivation and in vivo regarding uterine growth induction exhibiting a significant higher oral bioavailability than estradiol.

**Table 1**

| **Experimental readout** | **Example 1** | **Estradiol** |
|---|---|---|
| ERα binding [KF] | 1.6 | 1 |
| EC₅₀ ERα transactivation, U2OS cells [pM] | 82 | 15 |
| EC₅₀ ERα transactivation, MVLN assay [pM] | 1420 | 216 |
| EC₅₀ Alkaline phoshatase induction, Ishikawa cells [pM] | 759 | 72.6 |
| ED₅₀ Uterine growth, [µg/kg bodyweight] | 8.25 | 1.03 |
| Fmax (calculated from hepatocyte stability) | 34 % | 17% |

### Dosages

The present invention therefore provides a method of treating disorders mediated by an estrogen receptor in a subject in need thereof which comprises administering any of the compounds as defined herein in a quantity effective to treat said disorder. The compound may be administered to a patient by any conventional route of administration, including, but not limited to, intravenous, oral, subcutaneous, intramuscular, intradermal and parenteral. The quantity of the compound which is effective for contraception or treating a disorder mediated by an estrogen receptor is between 0.5 µg per kg and 1 mg per kg of subject body weight per day depending on route of administration, indication and potency of the respective compound.
The present invention also provides pharmaceutical compositions comprising one or more compounds of this invention in association with a pharmaceutically acceptable carrier. Preferably these compositions are in unit 30 dosage forms such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.025 to about 100 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.
The liquid forms in which the novel compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.
The method of treating a disorder mediated by an estrogen receptor described in the present invention may also be carried out using a pharmaceutical composition comprising any of the compounds as defined herein and a pharmaceutically acceptable carrier. The pharmaceutical composition may contain between about 5 mg and 1000 mg, preferably about 10 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.
Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen. For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and colouring agents can also be incorporated into the mixture. Suitable binders include, without limitation, starch, gelatine, natural sugars such as glucose or beta lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.
The liquid forms may include suitably flavoured suspending or dispersing agents such as the synthetic and natural gums, for example, tragacanth, acacia, methyl-cellulose and the like. For parenteral administration, sterile suspensions and solutions are desired. Isotonic preparations which generally contain suitable preservatives are employed when intravenous administration is desired.
The compound of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines. Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palmitoyl residue. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.
The compounds of this invention may be administered in any of the foregoing compositions and according to dosage regimens established in the art whenever contraception or treatment of a disorder mediated by an estrogen receptor is required. The daily dosage of the products may be varied over a wide range from 25 µg to 100 mg per adult human per day. For oral administration, the compositions are preferably provided in the form of tablets containing 0.025, 0.1, 0.5, 1.0, 5.0, 10.0, 15.0, 25.0, 50.0, 100 milligrams of the active ingredient for the symptomatic adjustment of the dosage to the woman in need of contraception or patient to be treated. Optimal dosages to be administered may be readily determined by those skilled in the art, and will vary with the particular compound used, the mode of administration, and the strength of the preparation and the advancement of the disease condition. In addition, factors associated with the particular patient being treated, including patient age, weight, diet and time of administration, will result in the need to adjust dosages.
The following examples are set forth to aid in the understanding of the invention, and are not intended and should not be construed to limit in any way the invention set forth in the claims which follow thereafter:
The compounds of the present invention can be prepared according to the following general schemes, using appropriate materials, and are further exemplified by the subsequent specific examples. The compounds illustrated in the examples are not, however, to be construed as forming the only genus that is considered as the invention. Those skilled in the art will readily understand that known variations of the conditions and processes of the following preparative procedures can be used to prepare these compounds. All temperatures are degrees Celsius unless otherwise noted.
   The final compounds of the present invention are synthesized as outlined in Schemes 1 to 8.

### GENERAL PROCESSES AND EXPERIMENTAL DETAILS

The new compounds of formula (I) are prepared according to the process outlined in scheme 1. Starting the synthesis with hydroxymethylation of 17β-hydroxy-5a-estr-1-en-3-ones **1** [US 19591229] with ethyl formiate under basic conditions [NL 6405235] the hydroxymethylated steroids of formula **2** are obtained. Reaction of these compounds with hydrazine gives 17β-hydroxy-5a-estr-1-eno[3,4-c]pyrazols **3.** Aromatization of such non aromatic pyrazolo-steroids under different conditions with several oxidizing agents, for example with palladium (II) hydroxide (US Patent 6399766) yields aromatic pyrazolo-steroids of formula **4.** Compounds with substituents in position 18 are synthesized in an analogous manner.

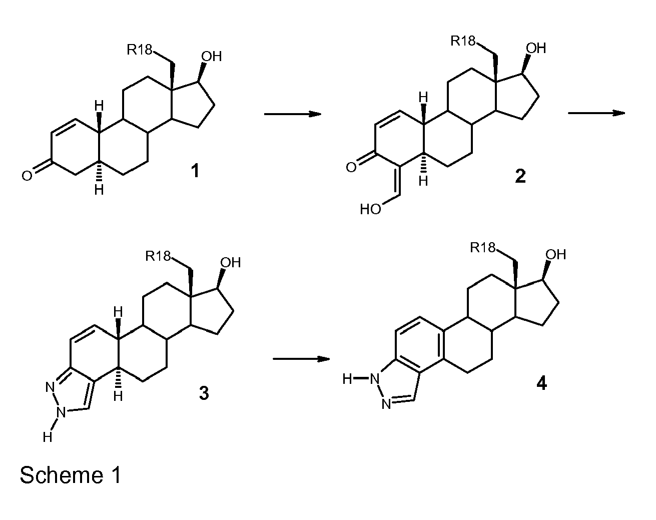

As an alternative the corresponding 5β-derivatives are also suitable as starting material in the same route as depicted in scheme 2. Starting the synthesis from the well described 17β-hydroxy-5β-estr-1-en-3-one **1a** (US 3007947) and following the synthesis as described above for scheme 1 the hydroxymethylation in position 4, pyrazole formation with hydrazine and finally an aromatization yield aromatic pyrazolo-steroids of formula **4**.

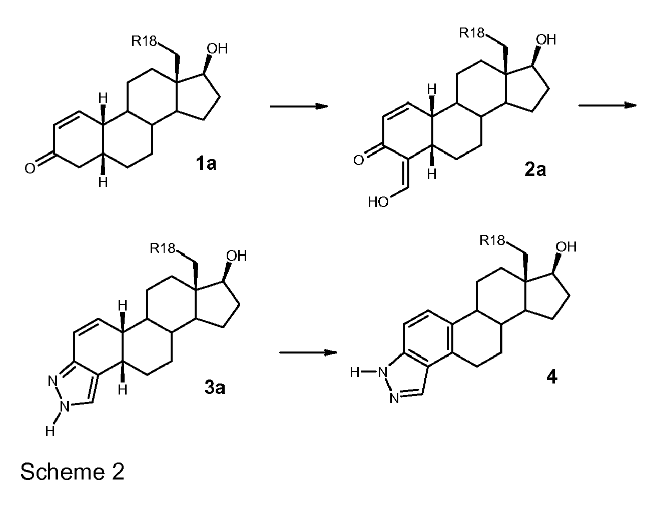

Substitutions in position 17 are achieved according to scheme 3. The sequence includes an oxidation of compound **4** with different oxidation agents or reactions known to an expert skilled in the art like Oppenauer oxidation, Dess-Martin periodinane (Dess.; Martin. J. Org. Chem. 48, 4155 (1983)) or with tetrapropylammonium-perruthenate / N-methyl-morpholine-n-oxide (Ley et al, Tetrahedron Lett. 30, 3204 (1989)) leading to the corresponding 17-ketone **5.** Reaction of these ketones **5** with different nucleophiles like oranomagnesium or arganolithium reagents gives 17-substituted pyrazolosteroids of formula **6.**

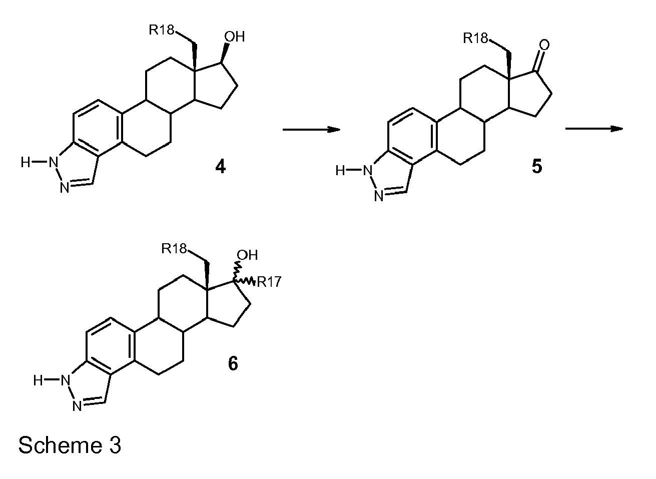

Alternatively, the synthesis starts from aromatic precursors, which can be prepared by methods known to an expert skilled in the art (see as a general reference: Fieser and Fieser: Steroids; Reinhold Publishing Cor. 1959), outlined in scheme 4 to give the pyrazolo-steroids **8.** The 4-formylestradiols **7** which can be prepared analogously according to the literature [Liu, Yong; Kim, Byoungmoo; Taylor, Scott D., Journal of Organic Chemistry (2007), 72(23), 8824-8830] can react with hydrazine under acidic conditions or directly with hydrazinehydrochloride in an analogous manner as described in the literature [Lokhande et al. Tetrahedron Letters; 48; 6890 (2007)].

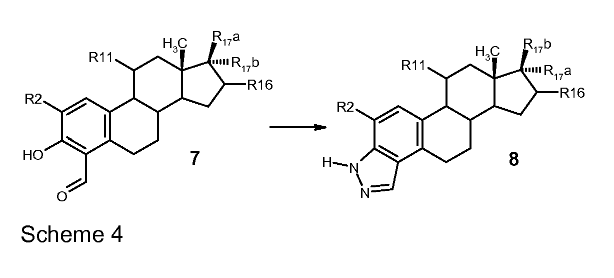

Substituents in position 5' can be introduced by the following sequence shown in scheme 5. Aldehydes of type **7** can be reacted with nucleophiles like Grignard reagents or organo lithium compounds to give alcohols of type **9**. These alcohols can be oxidized with different oxidizing agents (like chromium trioxide, tetrapropylammonium-perruthenate / N-methyl-morpholine-n-oxide) known to an expert in the field and give the ketones of type **10.** These can react with hydrazine hydrochloride in analogy to the procedure described in scheme 4 to give pyrazoles of type **11.**

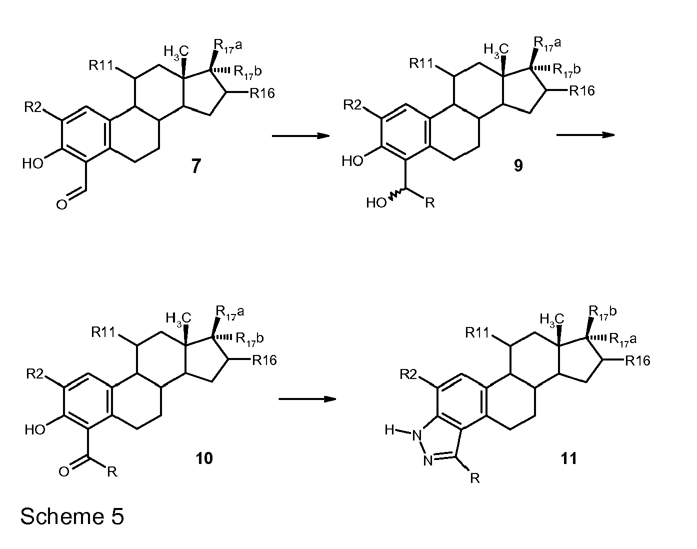

An additional approach is outlined in scheme 6. Starting from estradiol derivatives **12** 4-formyl-derivatives of type **13** can be made (J. Org. Chem. 72 (2007), 8824-30). The introduction of a 3-amino function can be achieved by making a 2-methyl-propionic-amide-ethers and subsequent treatment of the intermediate in a polar solvent. (see: J. Chem. Soc. 1990, 767-71; Org. Lett. 7, (2005), 3629-31). Cleavage of the propionic-acidanilide gives the free aniline **14.** An additional alternative for this transformation is the conversion of the phenol to a leaving group (e.g. triflate) and subsequent Palladium catalyzed reaction with nitrogen-containing compounds like benzylamin or sodium hexamethyldisilazane (see: Tetrahedron Lett. 44, (2003) 3071-73; Tetrahedron Lett. 46, (2005), 7111-15; J. Med. Chem. 49, (2006), 3832-49, Tetrahedron Lett. 43, (2002), 7617-20). The following transformations can be carried out in analogy to methods described in the literature (Org. Lett. 10, (2008), 1021-23). Hydroxylimin derivates **15** can be obtained after treatment with hydroxyamine. Subsequent dehydratization gives the desired pyrazoles **16.**

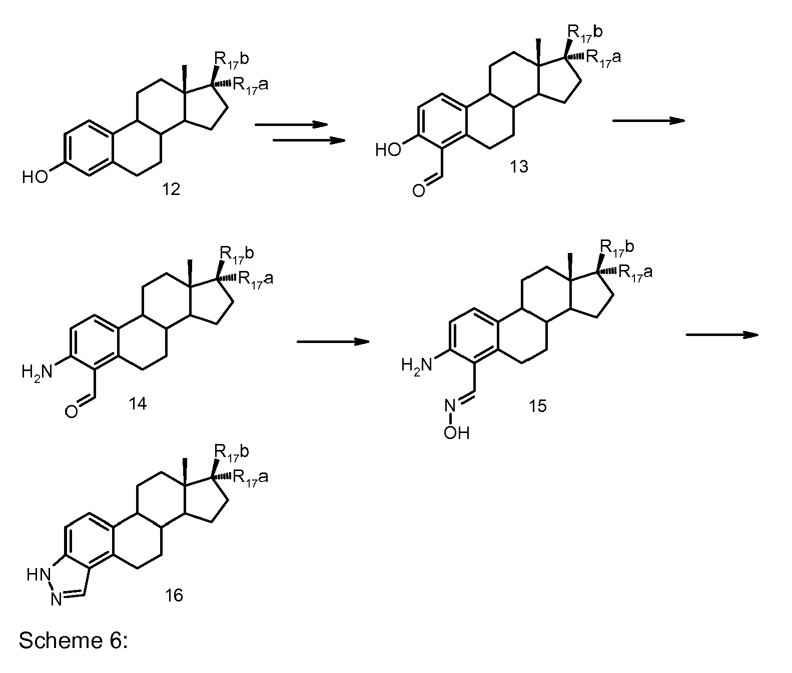

The triazoles can be prepared as outlined in scheme 7. A 4-nitro-estratrien derivative of type **17** (Horwitz et al., J. Med. Chem. 29, 692 (1986) can be transformed to a 3-amino derivative **18** in analogy to the process described above for scheme 6. The amino-nitro derivative **18** can be reduced to the corresponding diamino-derivative **19** by well known reduction methods.

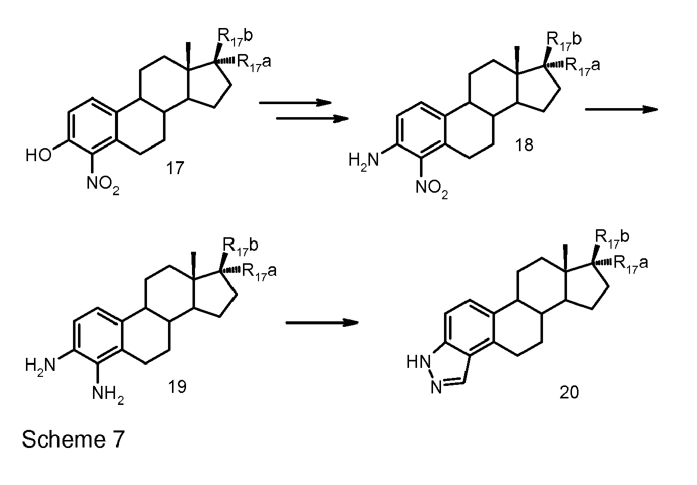

The triazoles **20** can be made by nitrosation for example with potassium nitrite and sulphuric acid (Chemische Berichte; 9; 222 (1876)).

### Example 1

### 1) 2'H-Pyrazolo[3',4':3,4]estra-1,3,5(10)-trien-17β-ol

### a) 17β-Hydroxy-4(Z)-hydroxymethylen-5α-estr-1-en-3-one

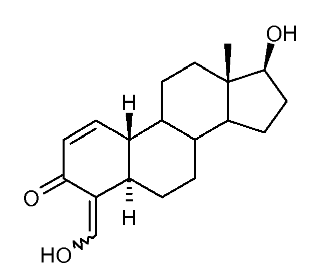

A solution of 500 mg 17β-hydroxy-5α-estr-1-en-3-one in 10 ml pyridine and 15 ml ethylformiate was cooled to -10°C. A solution of sodium methanolate (15 ml, 1M) was added in portions. The mixture was warmed to room temperature over two hours. The reaction mixture was poured into ice water and neutralized with hydrochloric acid, extracted with ethyl acetate, dried over sodium sulfate and concentrated in vacuo to yield 540 mg of yellow oil. This material was used in the next step without further purification and characterization.

### b) 2'H-Pyrazolo [5',4':3,4]-5α-estr-1-en-17β-ol

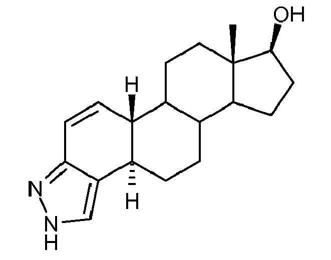

To a solution 520 mg of raw 17β-hydroxy-4(Z)-hydroxymethylen-5α-estr-1-en-3-one in 6 ml ethanol was added a solution of hydrazine in THF (1.9 ml, 1 M). The mixture was stirred at room temperature for three hours. The mixture was extracted with ethyl acetate and water. The organic phase was dried over sodium sulphate and concentrated in vacuo. The raw product was chromatographed on silica gel with hexane and ethyl acetate as eluents to yield 84 mg of.2'H-Pyrazolo [5',4':3,4]-5α-estr-1-en-17β-ol.

MS (CI+): m/z = 299 (M+1);
¹H-NMR (400 MHz, CDCl3): δ = 7.24 (s, 1H); 6.57 (d, 1H); 6.20 (d, 1 H); 3.71 (t, 1H); 2.45 (m, 1H); 1.05 - 2.50 (m, 16H); 0.76 (s, 3H)

### c) 2'H-Pyrazolo[3',4':3,4]estra-1,3,5(10)-trien-17β-ol

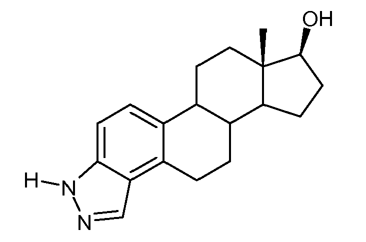

To a solution 1 g 2'H-Pyrazolo[5',4':3,4]-5α-estr-1-en-17β-ol of in 10 ml methanol were added 1.2 g of palladium hydroxide on charcoal (10 %). The mixture was heated in a sealed tube for 4 hours. The mixture was allowed to cool to room temperature, filtered over celite and concentrated in vacuo. The crude product was chromatographed on silica gel 60 with hexane/ethylacetate as eluent to give 64 mg of 2'H-Pyrazolo[3',4':3,4]estra-1,3,5(10)-trien-17β-ol as a white solid.

MS (CI+): m/z = 297 (M+H)⁺
¹H-NMR (400 MHz, CDCl3): δ = 8.03 (s, 1H); 7.39 (d, 1H); 7.30 (d, 1H); 3.76 (t, 1H); 3.15 (m, 2H); 2.39 (m, 2H); 2.16 (m, 1H); 2.01 (m, 2H); 1.72 (m, 1H); 1.20 - 1.60 (m, 7H); 0.81 (s, 3H)

### Example 2

### 2'H-Pyrazolo[3',4':3,4]estra-1,3,5(10)-trien-17-one

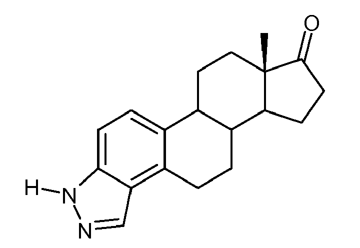

To a suspension of 521 mg of 2'H-Pyrazolo[3',4':3,4]estra-1,3,5(10)-trien-17β-ol (example 1) in 19 ml dichloromethane and 0.7 ml pyridine was added 746 mg of Dess-Martin periodinane. The reaction mixture was stirred for 2.5 hours at room temperature. The reaction mixture was diluted with water and extracted with ethyl acetate. The combined organic extracts were dried with sodium sulphate and concentrated in vacuo. The crude product was triturated with dichloromethane and hexane to give 325 mg of 2'H-Pyrazolo [3',4':3,4]estra-1,3,5(10)-trien-17-one as a white solid.

MS (CI+): m/z = 295 (M+H)⁺;
¹H-NMR (400 MHz, CDCl3): δ = 8.05 (s, 1 H); 7.39 (d, 1 H); 7.32 (d, 1 H); 3.22 (m, 2H); 2.50 (m, 3H); 1.92 - 2.23 (m, 4H); 1.45 - 1.80 (m, 5H); 0.94 (s, 3H); 0.88 (t, 1 H)

### Example 3

### 17α-Methyl-2'H-pyrazolo[3',4':3,4]estra-1,3,5(10)-trien-17β-ol

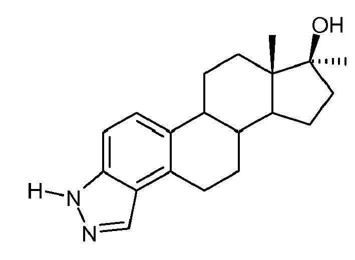

To a suspension of 80 mg of 2'H-Pyrazolo[3',4':3,4]estra-1,3,5(10)-trien-17-one in 10 ml of tetrahydrofuran were added 2.7 ml of methylmagnesiumbromide in tetrahydrofuran (3M). The reaction mixture was stirred for 20 hours. Then water was added and the mixture was extracted with ethyl acetate. The combined organic extracts were dried with sodium sulphate and concentrated in vacuo. The crude product was chromatoghraphed on silica gel 60 with hexane/ethyl acetate to yield 53 mg 17α-Methyl-2'H-pyrazolo[3',4':3,4]estra-1,3,5(10)-trien-17β-ol as a white solid.

MS (CI+): m/z = 311 (M+H)⁺;
¹H-NMR (400 MHz, CDCl3): δ = 8.03 (s, 1 H); 7.39 (d, 1 H); 7.30 (d, 1 H); 3.16 (m, 2H); 2.40 (m, 2H); 2.05 (m, 1 H); 2.01 (m, 2H); 1.20 - 1.80 (m, 8H); 1.30 (s, 3H); 0.92 (s, 3H)

## Claims

1. A compound of formula (I) 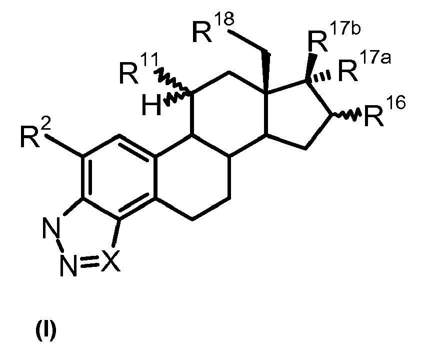 wherein
X is selected from the group consisting of nitrogen and CR^{a}, whereby
R^{a} stands for hydrogen, C₁₋₃-alkyl group, a CₚF₂ₚ₊₁ group with p = 1-3,
R² represents a hydrogen atom, a halogen atom, a C₁₋₃-alkyl group or a trifluoromethyl group,
R¹¹ is selected from the group of hydrogen, halogen, C₁₋₃-alkyl, C₂₋₃-alkenyl, C₂₋₃-alkinyl and C₁₋₃-alkoxy,
R¹⁶ is selected from the group consisting of hydrogen, hydroxyl, halogen, C₁₋₃-alkyl, C₂₋₃-alkenyl, C₂₋₃-alkinyl and trifluoromethyl,
R^{17a} and R^{17b} stand for a hydrogen atom, a hydroxyl group, an optionally substituted C₁₋₃-alkyl group, an optionally substituted C₂₋₃-alkenyl group, an optionally substituted C₂₋₃-alkinyl group, a halogen atom, a group -OCOR^{b}, whereby
R^{b} represents a group -(CH₂)ₙCOOH with n = 2 or 3, or a C₁₋₅-alkyl group with the proviso if R^{17a} stands for a hydroxyl group, R^{17b} represents a hydrogen atom or an optionally substituted C₁₋₃-alkyl group, an optionally substituted C₂₋₃-alkenyl group or a group -OCOR^{b} with the definition for R^{b} as mentioned above, and vice versa, or
R^{17a} and R^{17b} stand together for an oxygen atom,
and
R¹⁸ represents a hydrogen atom or a methyl group,
or a pharmaceutically acceptable salt thereof.

2. A compound according to Claim 1 wherein R^{17a} is selected from the group consisting of hydrogen, an optionally substituted C₁₋₃-alkyl group, an optionally substituted C₂₋₃-alkenyl group, an optionally substituted C₂₋₃-alkinyl group, whereas R^{17b} is selected from the group consisting of hydroxyl, fluorine, -OCOR^{b}.

3. A compound according to Claim 1, wherein R^{17a} is selected from the group consisting of hydrogen, methyl, trifluoromethyl, vinyl and ethinyl.

4. A compound according to Claim 1, wherein R^{17a} represents a hydroxyl group or a fluorine atom.

5. A compound according to Claim 1 wherein R¹⁶ is selected from the group consisting of hydrogen, hydroxyl and fluorine.

6. A compound according to Claim 1 wherein R¹⁸ is a hydrogen atom.

7. A compound according to claim 1, wherein X stands for CR^{a}.

8. A compound according to claim 1, wherein R^{a} stands for a hydrogen atom, a group trifluoromethyl or a methyl group.

9. A compound according to Claim 1, wherein R¹¹ represents a hydrogen atom, a fluorine atom, a hydroxyl group or a methoxy group.

10. A compound according to Claim 1, wherein R² stands for a hydrogen atom, a fluorine atom or a trifluoromethyl group. A pharmaceutical composition comprising a pharmaceutically acceptable carrier and a compound of Claim 1.

11. A compound according to claim 1, wherein R¹⁶ represents a hydroxyl group, R^{17a} a hydrogen atom and R^{17b} a fluorine atom.

12. A compound according to claim 1, wherein R^{17b} represents a hydroxyl group, R^{17a} a hydrogen atom, a vinyl, ethinyl, methyl or a trifluoromethyl group and R¹⁶ a hydrogen or fluorine atom or a hydroxyl group.

13. Compounds according to claim 1 to 12, namely
2'H-Pyrazolo[3',4':3,4]estra-1,3,5(10)-trien-17β-ol
17α-Methyl-2'H-pyrazolo[3',4':3,4]estra-1,3,5(10)-trien-17β-ol
17α-Ethyl-2'H-pyrazolo[3',4':3,4]estra-1,3,5(10)-trien-17β-ol
17α-Propyl-2'H-pyrazolo[3',4':3,4]estra-1,3,5(10)-trien-17β-ol
17α-Vinyl-2'H-pyrazolo[3',4':3,4]estra-1,3,5(10)-trien-17β-ol
17α-Ethinyl-2'H-pyrazolo[3',4':3,4]estra-1,3,5(10)-trien-17β-ol
2'H-Pyrazolo[3',4':3,4]estra-1,3,5(10)-trien-17-one
2-Fluoro-2'H-pyrazolo[3',4':3,4]estra-1,3,5(10)-trien-17β-ol
2-Fluoro-17a-methyl-2'H-pyrazolo[3',4':3,4]estra-1,3,5(10)-trien-17β-ol
17α-Ethyl-2-fluoro-2'H-pyrazolo[3',4':3,4]estra-1,3,5(10)-trien-17β-ol
2-Fluoro-17α-propyl-2'H-pyrazolo[3',4':3,4]estra-1,3,5(10)-trien-17β-ol
2-Fluoro-17α-vinyl-2'H-pyrazolo[3',4':3,4]estra-1,3,5(10)-trien-17β-ol
17α-Ethinyl-2-fluoro-2'H-pyrazolo[3',4':3,4]estra-1,3,5(10)-trien-17β-ol
11β-Fluoro-2'H-Pyrazolo[3',4':3,4]estra-1,3,5(10)-trien-17β-ol
11β-Fluoro-17α-Methyl-2'H-pyrazolo[3',4':3,4]estra-1,3,5(10)-trien-17β-ol
17α-Ethyl-11β-fluoro-2'H-pyrazolo[3',4':3,4]estra-1,3,5(10)-trien-17β-ol
11β-Fluoro-17α-Propyl-2'H-pyrazolo[3',4':3,4]estra-1,3,5(10)-trien-17β-ol
11β-Fluoro-17α-Vinyl-2'H-pyrazolo[3',4':3,4]estra-1,3,5(10)-trien-17β-ol
17α-Ethinyl-11P-fluoro-2'H-pyrazolo[3',4':3,4]estra-1,3,5(10)-trien-17β-ol
5'-Methyl-2'H-pyrazolo[3',4':3,4]estra-1,3,5(10)-trien-17β-ol
5',17-Dimethyl-2'H-pyrazolo[3',4':3,4]estra-1,3,5(10)-trien-17β-ol
2-Fluoro-5',17-dimethyl-2'H-pyrazolo[3',4':3,4]estra-1,3,5(10)-trien-17β-ol
2-Fluoro-5'-Methyl-2'H-pyrazolo[3',4':3,4]estra-1,3,5(10)-trien-17β-ol

14. Pharmaceutical composition comprising at least one compound of the general formula I according to any of Claims 1 to 13 and, optionally at least one additional active ingredient together with pharmaceutically suitable excipients and/or carriers.

15. Pharmaceutical composition according to claim 14, wherein the additional active ingredient is a SERM (selective estrogen receptor modulator) or a SERD (selective estrogen receptor destabilizer).or a progestogen.

16. A process for making a pharmaceutical composition comprising mixing a compound of Claim 1 and optionally at least one additional active ingredient with a pharmaceutically acceptable carrier.

17. Use of compounds according to claim 1 to 13 for manufacturing a medicament for the treatment of a disorder mediated by an estrogen receptor, in a subject in need thereof comprising administering to the subject a therapeutically effective amount of the compound of Claim 1.

18. Use according to Claim 16, wherein the disorder mediated by an estrogen receptor is selected from the group consisting of hot flashes, vaginal dryness, osteopenia, osteoporosis, hyperlipidemia, loss of cognitive function, degenerative brain diseases, cardiovascular diseases, cerebrovascular diseases, cancer of the breast tissue, hyperplasia of the breast tissue, cancer of the endometrium, hyperplasia of the endometrium, cancer of the cervix, hyperplasia of the cervix, cancer of the prostate, benign prostatic hyperplasia, endometriosis, uterine fibroids and osteoarthritis.

19. Use according to Claim 16, wherein the disorder mediated by an estrogen receptor is selected from the group consisting of osteoporosis, hot flashes, vaginal dryness, breast cancer and endometriosis.

20. Use of compounds according to claim 1 to 13 for manufacturing a medicament for the treatment of a disorder mediated by an estrogen receptor in a subject in need thereof comprising administering to the subject a therapeutically effective amount of the composition of Claim 14.

21. Use of compounds according to claim 1 to 13 for contraception, alone or in combination with an effective amount of a compound as in Claim 1 to 13 and a progestogen.
